# EUROPÄISCHE PATENTSCHRIFT

(11) **EP 3 405 221 B1**
(45) Veröffentlichungstag und Bekanntmachung des Hinweises auf die Patenterteilung: **15.04.2020**
(21) Anmeldenummer: 16836223.4
(22) Anmeldetag: 28.12.2016
(51) Int. Cl.: A61L 27/06, A61L 27/10, A61L 27/30, A61F 2/34

(54) **ORTHOPÄDISCHES IMPLANTAT**
ORTHOPAEDIC IMPLANT
IMPLANT ORTHOPÉDIQUE

(30) Priorität: 29.12.2015 DE 102015016894
(43) Veröffentlichungstag der Anmeldung: 28.11.2018
(73) Patentinhaber: ZM Präzisionsdentaltechnik GmbH, 18055 Rostock (DE)
(72) Erfinder: MITROVIC, Milija, 1805 Rostock (DE)
(86) Internationale Anmeldenummer: PCT/DE2016/000442
(87) Internationale Veröffentlichungsnummer: WO 2017/114520

(56) Entgegenhaltungen:
- WO-A1-2012/010899
- DE-T2- 60 306 739
- ZOTHNER A ET AL: "Die Evolution des Abutments", QUINTESSENZ DER ZAHNTECHNIK, QUINTESSENZ VERLAGS, DE , Bd. 35, Nr. 5 1. Januar 2009 (2009-01-01), Seiten 2-16, XP002676248, ISSN: 0340-4641 Gefunden im Internet: URL:http://www.memodent.nl/upload/content/ documents/qz_05_2009_zothner.pdf
- DATABASE WPI Week 199331 Thomson Scientific, London, GB; AN 1993-247613 XP002769561, & JP H05 168693 A (KYOCERA CORP) 2. Juli 1993 (1993-07-02)
- ANIKA JONITZ-HEINCKE ET AL: "The Impact of Metal Ion Exposure on the Cellular Behavior of Human Osteoblasts and PBMCs: In Vitro Analyses of Osteolytic Processes", MATERIALS, vol. 10, no. 734, 3 July 2017 (2017-07-03) , pages 1-15, XP055642327, DOI: 10.3390/ma10070734

## Beschreibung

Die Erfindung betrifft ein orthopädisches Implantat für den Ersatz von Gelenken, mit einem Einsatzkörper aus einem nichtmetallischen Material als Keramik und mit einer diesen zumindest teilweise umgebenden metallischen Pfanne aus Titanwerkstoffen.

Unter anderem kann es sich bei dem Implantat um einen Teil einer Hüftgelenk-Endoprothese handeln, bei der eine femoralen Komponente in den Röhrenknochen des Oberschenkels einführbar ist und bei der eine Schale auf der acetabulären Seite als Gleitpartner dient. Bei letzterer wird üblicherweise in einer Pfanne aus einem metallischen Werkstoff, die den Kontakt zum körpereigenen Knochengewebe vermittelt, ein Einsatzkörper aus einem nichtmetallischen Material angeordnet.

Bei den heute in der überwiegenden Mehrzahl der Fälle, insbesondere für Hüftgelenk-Endoprothesen, bestehen die dabei verwendeten Einsatzkörper zumeist aus einem - unter Umständen hochvernetzten oder XPE (cross-linked) - Polyethylen-Material und stellen eine der wichtigsten Ursachen für eine Prothesenlockerung mit einer durch Abriebpartikel hervorgerufenen Entzündungsreaktion der Gelenke dar. Andererseits bestehen aber auch bei Metall/Metall-Gleitpaarungen, beispielsweise auf der Basis von Kobalt-Chrom- oder Edelmetall-Werkstoffen, wie sie daneben verwendet werden, durch die Freisetzung von Metallionen erhebliche Risiken. Um derartige Komplikationen weitgehend auszuschließen, stellt es eine bereits bekannte Maßnahme dar, bei einem Implantat der eingangs genannten Art ein nichtmetallisches Material als Einsatzkörper zu verwenden, also beispielsweise bei den bereits erwähnten Hüftgelenk-Endoprothesen einen nichtmetallischen Einsatzkörper in eine metallische Pfanne, die dann beispielsweise aus einem Titanwerkstoff besteht, einzusetzen. Speziell kann auf diese Weise beispielsweise mit einer in einem Plasmasprayverfahren aufgebrachten Beschichtung aus einem porösen Calciumphosphat bzw. Titan auf die äußere Oberfläche der Pfanne eine gute Fixierung mit dem umgebenden Knochengewebe erzielt werden. In diesem Zusammenhang ist es auch eine bereits bekannte Maßnahme, eine solche Titanpfanne mit Durchgangsöffnungen zu versehen.

Keramiken stellen bei Gelenkimplantaten ein zunehmend beliebtes und genutztes Material dar, wobei bei lasttragenden Oberflächen aufgrund ihrer hervorragenden tribologischen Eigenschaften häufig Aluminiumoxid, Zirkonoxid oder Mischungen aus diesen verwendet werden. Darüber hinaus zeichnen sich diese Materialien durch eine gute Biokompatibilität und durch eine hohe Akzeptanz im menschlichen Körper aus. Jedoch besteht bei diesen Materialien in der Regel nur eine verhältnismäßig geringe Neigung einer Osseointegration, auch ist ihre Flexibilität nicht die gleiche wie bei einem Titanwerkstoff. Aus diesem Grund wurden bereits eine Vielzahl von Oberflächenmodifikationen und Beschichtungen für dentale und orthopädische Implantate vorgeschlagen, bei denen ein Titan-Grundkörper mit einem Coating bzw. einer Oberflächenstrukturierung versehen wird. Dabei kommen auch Beschichtungen aus Zirkonoxid und bioaktiven Gläsern zum Einsatz. Eine besonderes Augenmerk gilt dabei der Möglichkeit einer Revisionsoperation, also, falls erforderlich werdend, einer Explantation eines zuvor eingesetzten Implantates.

Nach der DE 603 06 739 T2 ist bereits eine Hüftprothese mit keramischen Einsatzkörper bekannt. Hierbei wird die Verbindung mit einer metallischen Pfanne aus Titanwerkstoff einer weichen Metallzwischenschicht durchgeführt. Es wird dabei keine keramikartige Oberfläche gebildet, die eine Basis für einen nachfolgenden Lötvorgang ermöglicht.

Aufgabe der Erfindung ist es, ein Implantat der eingangs genannten Art derart auszubilden, dass sich dieses einerseits gut mit dem umgebenden Knochenmaterial im Sinne einer Osseointegration verbindet und dass das Implantat zugleich ohne Schwierigkeiten auch eine Revisionsoperation ermöglicht.

Die Erfindung löst diese Aufgabe, indem sie vorsieht, dass die Fläche der metallischen Pfanne mit einem in einem keramischen Brand verfestigten silikatischen Glaslot vorbeschichtet ist und seine anschließende Verlötung mit der Außenfläche des Einsatzkörpers über ein aus einem auf Siliziumdioxid (SiO₂) basierendes Glaslot erfolgt, das die beiden Bestandteile miteinander verbindet und die metallische Pfanne mit Durchbrechungen in Form einer Vielzahl kleiner Löcher als Netzstruktur bzw. als Lochblech ausgebildet ist.

Ein solches Implantat stellt eine biokompatible Lösung für sämtliche Gelenke des menschlichen Körpers dar, also insbesondere für künstliche Knie-, Hüftkugel-, Schulteroder Fingerkuppengelenke; auch können Komponenten für die Wirbelsäule, für andere kleine Gelenke aus den erfindungsgemäßen Implantaten gefertigt werden. Als besonders geeignet für die Herstellung solcher Implantate haben sich dabei generative Fertigungsverfahren wie beispielweise das Rapid-Prototyping erwiesen. Der in die metallische Pfanne eingesetzte Keramikkörper kann sowohl aus einer reinen Aluminiumoxidkeramik, einer Zirkonoxidkeramik oder aber aus einer sogenannten Mischoxidkeramik aus Aluminium- und Zirkonoxid, gegebenenfalls mit einem Zusatz aus Yttriumoxid, bestehen.

Die vollständige Beschichtung einer durchbrochenen Netzstruktur aus einer Titanlegierung, die dadurch keramische Eigenschaften annimmt, eignet sich vor allem für ein zementfreies Implantat, bei dem eine sichere Abdeckung und das Einfangen aller aus dem Verband herausgelösten Metallionen, in diesem Fall vorwiegend von Titanoxiden, gewährleistet ist. Bei einem solchen vollkeramischen Netz wird die Verbindung mit dem darunter angeordneten Keramikkörper über ein Glaslot hergestellt, das in der Lage ist, zwei Keramiken miteinander zu verbinden. Die Vorbehandlung erfolgt mittels eines Verfahrens, bei dem eine Schicht aus einem Keramiklot gleichmäßig aufgesprüht und anschließend einem Brennprozess unterzogen wird. Durch diesen Brennprozess erhält das Metall eine keramikartige Oberfläche und es werden nicht nur sämtliche freigesetzten Titanoxide gebunden, sondern es wird gleichzeitig die Rauigkeit der Trtanoberfläche ausgeglichen und damit eine optimale Basis für den nachfolgenden Lötvorgang gebildet.

Nachfolgend soll die Erfindung anhand eines in der Zeichnung dargestellten Ausführungsbeispiels näher erläutert werden. Es zeigen:
- Fig.1: einen Keramikkörper für eine Hüftgelenk-Endoprothese in perspektivischer Darstellung,
- Fig.2: und 3 zwei verschiedene Ausführungsformen einer ursprünglich metallischenPfanne, die den keramischen Einsatzkörper umgibt und die jeweils mit einer Vielzahl von kleinen Öffnungen versehen ist,
- Fig.4: das Zusammenfügen eines keramischen Einsatzkörpers mit der Netzstruktur,
- Fig.5: eine schematische Darstellung direkt zugeordneter Pfanne und Einsatzkörper und
- Fig.6: eine Darstellung gemäß Fig. 5 mit Abstandhaltern zwischen Pfanne und Einsatzkörper.

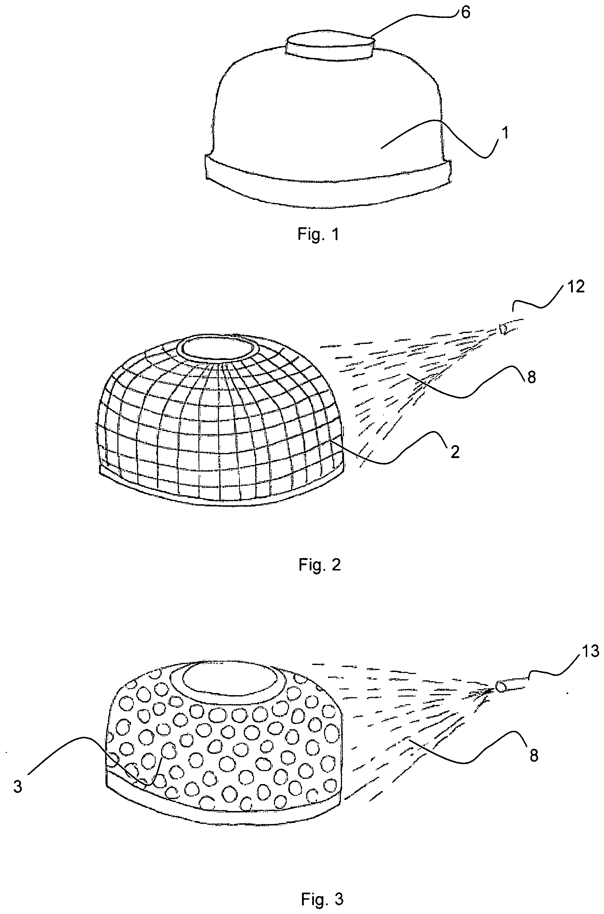

Die Darstellung gemäß der Fig. 1 zeigt einen keramischen Einsatzkörper 1 für eine Hüftgelenk-Endoprothese, der in eine ursprünglich als metallische Füanne oder Schale 2, 3 ausgebildeten Hülle eingesetzt wird. Der Einsatzkörper 1 besteht entweder aus einer reinen Aluminiumoxidkeramik, einer Zirkonoxidkeramik oder aus einer sogenannten Mischoxidkeramik aus Aluminium- und Zirkonoxid, gegebenenfalls mit einem Zusatz aus Yttriumoxid, und ist mit einem einstückig angeformten zentrischen Stopper 6 versehen, der sich durch eine zentrische Durchgangsbohrung der Schale 2, 3 hindurch erstreckt und der unter anderem dem Abfluss überschüssigen Lotes dient. Bei dem Werkstoff der Schale 2, 3 handelt es sich im Falle der hier dargestellten Ausführungsbeispiele der Erfindung ursprünglich um die Titanlegierung Ti-6Al-4V (Titanium Grade 5).

Die Titanschale weist im Falle der in den Figuren 2 bis 4 dargestellten Ausführungsbeispielen der Erfindung eine Vielzahl kleiner Öffnungen auf und kann als Netzstruktur, wie in Fig. 2 dargestellt, oder nach Art eines dreidimensionalen Lochbleches 3 gemäß Fig. 3 ausgebildet sein. Mittels Airbrush-Technik 12, 13 oder mit Hilfe einer anderen geeigneten Technik wird eine dünne, aber deckende Schicht eines silikatischen Glaslotes 8 auf die Struktur aufgetragen und diese wird anschließend einem keramischen Brand unterzogen. Wichtig ist dabei, dass sämtliche Bereiche der Titanschale 2, 3 mit dem Lot 8 beschichtet sind und einem keramischen Brand unterzogen werden, der dann eine tropfenfreie, gleichmäßige Schicht erzeugt, die verfestigt ist.

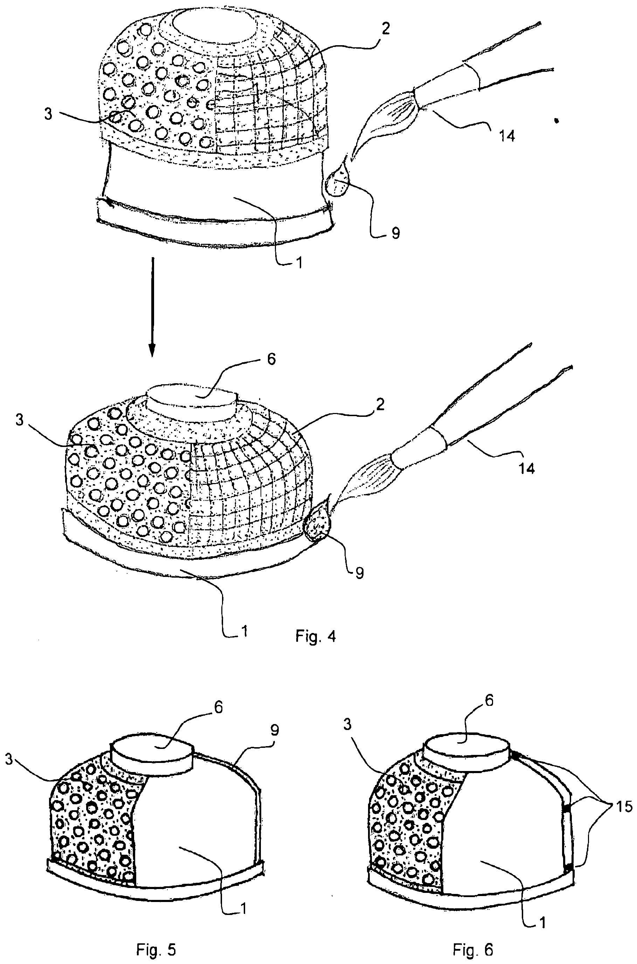

Danach wird die Schale 2, 3 entweder direkt oder über Abstandhalter 15 auf den keramischen Einsatzkörper 1 gesetzt und mit diesem durch ein Glaslot 9 auf der Basis von Si0₂, Al2O3, K₂0 und Na₂0 in einem Lötvorgang verbunden, bei dem die Temperatur vorzugsweise unter 850 °C gehalten wird, um einen Phasenübergang des Titans zu vermeiden. Vor diesem Lötvorgang werden die Stege und die Innenränder der Netz- bzw. der Lochblechstruktur 2, 3 mit dem Glaslot 9 bedeckt, beispielsweise mit einem Pinsel 14, wie dies in der Anordnung gemäß Fig. 4 dargestellt ist. Der Abstand der beiden Teile, d. h., des Einsatzkörpers 1 und der Titanstruktur 2 bzw. 3, zueinander, ergibt sich dabei aus der Schichtdicke des auf die besagten Teile der Titanstruktur 2, 3 und auf die Außenfläche des keramischen Einsatzkörpers 1 aufgetragenen Glaslotes 9. Die durch diesen Lötvorgang erzeugte Verbindung zwischen dem Einsatzkörper 1 und der Struktur der Titanschale 2 bzw. 3 eignet sich nunmehr für eine zementfreie Fixierung des Implantates im Körper eines Patienten.

Die Geometrie einer knie-endoprothetischen Gleitpaarung ist im Prinzip zwar wesentlich komplexer als diejenige eines Hüftgelenkes, jedoch können auch in diesem Fall Implantate der vorangehend beschriebenen Art hergestellt und verwendet werden. Auch können Komponenten für die Wirbelsäule, für kleine Gelenke aus solchen Implantaten gefertigt werden. Entscheidend ist in jedem Fall, dass die Titanstruktur zunächst mithilfe eines silikatischen Glaslotes keramische Eigenschaften annimmt, so dass alle metallischen Abriebpartikel sicher eingeschlossen werden, insbesondere keine Titan Ionen austreten können und später höchstens Siliziumabrieb zu finden ist, wobei die Verbindung zwischen dem keramischen Einsatzkörper und der ebenfalls "keramischen" Titanpfanne mittels eines silikatischen Glaslotes erfolgt, das geeignet ist, beide keramischen Bestandteile miteinander zu verbinden.

## Patentansprüche

1. Orthopädisches Implantat für den Ersatz von Gelenken, mit einem Einsatzkörper aus einem nichtmetallischen Material als Keramik und mit einer diesen zumindest teilweise umgebenden metallischen Pfanne aus Titanwerkstoffen, **dadurch gekennzeichnet, dass** die Fläche der metallischen Pfanne (2, 3) mit einem in einem keramischen Brand verfestigten silikatischen Glaslot vorbeschichtet ist und seine anschließende Verlötung mit der Außenfläche des Einsatzkörpers (1) über ein aus einem auf Siliziumdioxid (SiO₂) basierendes Glaslot (9) erfolgt, das die beiden Bestandteile miteinander verbindet und die metallische Pfanne (2,3) mit Durchbrechungen in Form einer Vielzahl kleiner Löcher als Netzstruktur (2) bzw. als Lochblech (3) ausgebildet ist.

2. Implantat gemäß Anspruch 1, **dadurch gekennzeichnet, dass** der Einsatzkörper (1) aus einer Keramik auf der Basis von Aluminiumoxidkeramik besteht.

3. Implantat gemäß Anspruch 1, **dadurch gekennzeichnet, dass** der Einsatzkörper (1) aus einer Keramik auf der Basis von Zirkonoxidkeramik besteht.

4. Implantat gemäß Anspruch 1, **dadurch gekennzeichnet, dass** der Einsatzkörper (1) aus einer Keramik auf der Basis von Mischoxidkeramik besteht.

5. Implantat gemäß einem der Ansprüche 1 oder 2, **dadurch gekennzeichnet, dass** die Pfanne (2, 3) aus Titan besteht.

6. Implantat gemäß einem der Ansprüche 1 oder 2, **dadurch gekennzeichnet, dass** die Pfanne (2, 3) aus einer Titanlegierung besteht.

7. Implantat gemäß einem der Ansprüche 1 bis 6, **dadurch gekennzeichnet, dass** zwischen metallischer Pfanne (2, 3) und Einsatzkörper (1) Abstandshalter (15) vorgesehen sind.

## Claims

1. An orthopaedic implant for the replacement of joints, with an insert body made of a non-metallic material as ceramic and with a metallic socket made of titanium materials that at least partially surrounds the insert body, **characterised in that** the surface of the metallic socket (2, 3) is pre-coated with a silicate glass solder that is hardened in a ceramic firing, and its subsequent soldering with the outer surface of the insert body (1) is achieved via a glass solder (9) based on silicon dioxide (SiO₂), which joins the two components to one another, and the metallic socket (2, 3) is embodied as a net structure (2) or as a perforated metal sheet (3), having through-apertures in the form of a plurality of small holes.

2. The implant according to claim 1, **characterised in that** the insert body (1) consists of a ceramic based on aluminium oxide ceramic.

3. The implant according to claim 1, **characterised in that** the insert body (1) consists of a ceramic based on zirconium oxide ceramic.

4. The implant according to claim 1, **characterised in that** the insert body (1) consists of a ceramic based on mixed oxide ceramic.

5. The implant according to one of claims 1 or 2, **characterised in that** the socket (2, 3) consists of titanium.

6. The implant according to one of claims 1 or 2, **characterised in that** the socket (2, 3) consists of a titanium alloy.

7. The implant according to one of claims 1 to 6, **characterised in that** spacers (15) are provided between the metallic socket (2, 3) and insert body (1) .

## Revendications

1. Implant orthopédique pour le remplacement d'articulations, comprenant un corps d'insertion en matériau non-métallique en tant que céramique et une coupelle métallique en matériaux de titane entourant celui-ci au moins partiellement, **caractérisé en ce que** la surface de la coupelle métallique (2, 3) est pré-revêtue par un verre à soudure silicaté solidifié par cuisson céramique et son soudage consécutif avec la surface extérieure du corps d'insertion (1) est effectué par un verre à soudure (9) à base de dioxyde de silicium (SiO₂), qui relie entre elles les deux composantes, et la coupelle métallique (2, 3) est formée avec des évidements sous forme d'une pluralité de petits trous en tant que structure de filet (2), respectivement, tôle perforée (3).

2. Implant selon la revendication 1, **caractérisé en ce que** le corps d'insertion (1) est composé d'une céramique à base de céramique d'oxyde d'aluminium.

3. Implant selon la revendication 1, **caractérisé en ce que** le corps d'insertion (1) est composé d'une céramique à base de céramique d'oxyde de zircon.

4. Implant selon la revendication 1, **caractérisé en ce que** le corps d'insertion (1) est composé d'une céramique à base de céramique d'oxyde mixte.

5. Implant selon l'une des revendications 1 ou 2, **caractérisé en ce que** la coupelle (2, 3) est en titane.

6. Implant selon l'une des revendications 1 ou 2, **caractérisé en ce que** la coupelle (2, 3) est en alliage de titane.

7. Implant selon l'une des revendications 1 à 6, **caractérisé en ce que** des écarteurs (15) sont prévus entre la coupelle métallique (2, 3) et le corps d'insertion (1).
